Europäisches Patentamt

European Patent Office

Office européen des brevets

(19)

(11) Publication number: 0 178 791
A1

(12) # EUROPEAN PATENT APPLICATION

(21) Application number: 85306573.8

(22) Date of filing: 16.09.85

(51) Int. Cl.⁴: G 01 N 33/545

(30) Priority: 14.09.84 GB 8423228

(43) Date of publication of application:
23.04.86 Bulletin 86/17

(84) Designated Contracting States:
AT BE CH DE FR GB IT LI NL SE

(71) Applicant: UNILEVER PLC
Unilever House Blackfriars P.O. Box 68
London EC4P 4BQ(GB)

(84) Designated Contracting States:
GB

(71) Applicant: UNILEVER NV
Burgemeester s'Jacobplein 1 P.O. Box 760
NL-3000 DK Rotterdam(NL)

(84) Designated Contracting States:
BE CH DE FR IT LI NL SE AT

(72) Inventor: Bosley, John Anthony
10 Philip Way Higham Ferrers
Wellingborough Northants NN9 8LG(GB)

(72) Inventor: Watkins, Neil David
2 Baddesley Road Olton
Solihull West Midlands B92 7LP(GB)

(74) Representative: Stancliffe, Terence Christopher et al,
UNILEVER PLC Patents Division P.O. Box 68 Unilever
House
London EC4P 4BQ(GB)

(54) Specific binding materials, their preparation and use.

(57) A process for producing a polymeric product with specific binding affinity, for an antigen or antibody or member of another specific binding pair, comprising the steps of (a) forming a covalently-linked reaction product which is a derivative both of (1) a member of a specific binding pair and of (2) a polymerisable monomer, and (b) polymerising the reaction product from step (a) as a minor component of a polymerisable comomomer mixture.

EP 0 178 791 A1

Croydon Printing Company Ltd

Specific binding materials, their preparation and use

This invention relates to improvements in specific binding materials, their preparation and use. The invention also relates to novel intermediate materials specially suited to the production of the improved specific binding materials.

Specific binding effects are well known as those resulting especially from the strong specific affinities of antigens and haptens for their individual antibodies, and from the specific affinities of certain other pairs of biological materials, such as hormones and their corresponding hormone-binding proteins from serum, and the specific affinity of biotin for avidin or streptavidin, for example.

Specific binding materials have been widely described in the prior art, comprising the combination of one member of some particular specific binding pair, with a solid base material. The methods of combination and the base materials vary very widely. For example, some known specific binding materials comprise a protein member of a

- 2 -                    P3009 0178791

specific binding pair physically adsorbed to a clean plastics surface, e.g. polystyrene, as for example the inside surface of a microlitre well.  Alternatively, a wide variety of covalent bonding techniques have been applied to the production of specific binding materials, involving the chemical derivatisation of many solid substrates, including plastics, glass, polysaccharides and lipids.

Chemical derivatisation procedures, though in some cases capable of assuring firm bonding of the specific binding pair member to the substrates, often suffer from poor reproducibility and precision, from limited rates of incorporation or derivatisation, and often involve the use of highly toxic materials.

A prior art search has also disclosed the following:-

GB 2 114 287 (Otsuka) discloses binding reactions of "TAG-like material" (a class including sulfated glycoprotein of porcine gastric mucosa) (which can bind to plant-derived lectins) which is rendered insoluble, in a form of binding assay analogous to sandwich immunoassays, using plant-derived lectins (binding proteins). GB 2 114 287 discloses radiation-induced polymerisation of aqueous monomer dispersion containing TAG or TAG-like material or a specific lectin, so that the TAG-like material is included in the resulting polymer matrix, and in particular a polymeric preparation made by polymerising a dispersion solution of sulphates glycoprotein of porcine gastric mucosa (PGM) with hydroxyethylmethacrylic acid. It is believed that in this product the PGM is occluded within an acrylic polymer matrix.  The specification does not disclose or suggest the derivatisation of specific binding materials by covalent linkage to polymerisable monomers.

- 3 -                    P3009 0178791

GB 1 190 384 (Murphy) discloses 2,5-dichloro-4,6-dinitrophenyl esters of acrylic, alpha-methacrylic, crotonic and 3-methyl crotonic acid as examples of fungicidal dinitrodihalophenyl esters.

GB 909 372 (Hoechst) mentions the acrylic ester of 2,4-dinitro-6-sec-butyl phenol as a herbicidally active substance.

GB854 442 (Rohm and Haas) discloses methacrylates of nitrophenyl compounds as miticides and fungicides.

GB 1 420 509 (Sumitomo) discloses intermediates in the synthesis of biotin, including esters of N-substituted carboxybiotin (Formula X).

GB 1 290 944 (Hoffmann la Roche) discloses the synthesis of alpha-dehydrobiotin, as an antibiotic against bacteria and fungi.

GB 1 252 336 (Cancer Institute Board) discloses production of graft copolymers containing substituted polystyrene graft polymer portions which are subsequently derivatised with steroids.

GB 1 550 151 and 1 550 465 (Kansai) describe the immobilisation of enzymes and microbial cells in polymer matrices by mixing cells or enzymes with photocurable resin and then polymerising the mixed preparations.

GB 2 071 669 (Italfarmaco) describes the immobilisation of enzymes on polysaccharide matrices by using small quantitates of bifunctional vinyl monomer as coupling agent.

EP 77 671 (Ortho) describes conjugates of immunologically-related substances with polymer made by linking the substances to polyacrylamide polymers via avidin-biotin linkage: and also describes the production of fluorescent polymer by making a fluorescent monomer from fluoresceinamine and acryloyl chloride and copolymerising the reaction product with acrylic acid and acrylamide: subsequently the polymers produced are described as activated for later linkage of antibody thereto; in order to give antibody labelled with fluorescent polymer.

An aim of this invention is to provide processes for providing specific binding materials bonded to solid substrates with good reproducibility and precision.

Another aim of the invention is to provide high levels of incorporation of the specific binding materials.

Another aim of the invention is to limit the need to use highly toxic materials in such preparation.

According to the invention we provide a process for producing a polymeric product with specific binding affinity for an antigen or antibody or member of another specific binding pair, comprising the steps of (a) forming a covalently linked reaction product which is a derivative of both (1) a member of a specific binding pair and (2) a polymerisable monomer, and (b) polymerising the reaction product from step (a) as a minor component of a polymerisable comonomer mixture.

A product according to the invention can be for example a polymeric product comprising specific binding material, (provided by the invention both separately and in association with its specific binding partner forming a specific binding pair), which comprises a polymeric solid material which has been produced by copolymerisation of a major monomeric constituent together with a copolymerisable derivative of a member of the specific binding pair to confer on the polymer a specific binding reactivity at its solid surface.

In this specification, "polymeric" and similar words refer not only to polymers in the strictest sense, such as polymeric vinyl products, but also for example to analogous products of condensation processes capable of yielding high-m.w. products from low-m.w. starting materials.

Used according to the invention are polymerisable derivatives of members of specific binding pairs, and polymerisable monomer mixtures including such polymerisable derivatives as comonomers, especially in relatively minor amount, e.g. under 5%, e.g. under 1% or even less than 0.5% or less than 0.1%. Also provided by the invention are specific binding assay procedures and other affinity reaction procedures in which the polymeric products are used, and bound complexes including such polymeric products and their specific binding partners in the bound state.

In preferred examples, those monomers can be used which can be polymerised in aqueous solution by free-radical polymerisation processes. Notable and preferred examples are acrylic monomers such as acrylic acid, acrylamide, methacrylic acid and any of their polymerisable esters and derivatives especially including crosslinker derivatives such as methylene-bis-acrylamide. The techniques and monomers used in the polymerisation, apart from the specific binding derivatives described herein, can be chosen widely from those already known and used.

Also within the scope of the invention are polymeric specific binding materials based on any other polymerisation process capable of taking place in conditions compatible with particular specific binding material chosen. In some cases this means that dilute aqueous solution is the preferred polymerisation medium. An alternative to free radical polymerisation can be anionic or cationic or ring-opening polymerisation, e.g. of ethylene oxide and/or propylene oxide and other 1,2-epoxy ethyl compounds and derivatives.

The preferred specific binding materials to be made the basis of copolymerisable comonomers are low-molecular weight specific binding materials, (e.g. of molecular weight up to about mw 1500), such as haptens, steroids, antibiotics, or (e.g.) biotin, which will be used later in conjunction with their corresponding antibodies. One notably useful specific binding material for use in the invention is biotin or a derivative thereof, to be used later in conjunction with its specific binding partner avidin or streptavidin. Another useful specific binding material is 2,4-dinitrophenol or a derivative thereof, especially a medium- or long-chain derivative thereof, to be used later in conjunction with its specific binding partner anti-DNP antibody. Further examples of suitable specific binding materials are progesterone or another steroid, such as pregnanediol glucuroride or estrone glucuronide, or gentamicin or another antibiotic, e.g. a saccharide antibiotic, in each case to be used later in conjunction with its available specific antibody binding partner.

Biotin and other specific binding materials containing carboxyl groups can in many examples be derivatised for coupling to suitable polymerisable monomers by intermediate formation of a suitable acid halide or ester, amide or hydrazide, and then substituting the intermediate derivatising group by a polymerisable derivative, e.g. a hydroxyl-containing polymerisable material such as a hydroxyalkyl ester of acrylic or methacrylic acid.

Alternatively, an acid derivative of a polymerisable carboxyacid, e.g. acryloyl chloride, or a methacryloyl halide, or compounds of equivalent acylating activity, can be used to derivatise an amino or hydroxy group in a specific binding material or derivative thereof. Many known forms of coupling reaction can be chosen for use in preparing derivatives of a specific binding compound and a polymerisable compound. For example, the two routes mentioned can be combined by first esterifying biotin with (excess) polyethylene glycol via the acid halide, followed by reaction of the monoester product with acryloyl halide or acylating derivative of another acrylic monomer. (These ester-forming reactions can be carried out especially for example in benzene or toluene with triethylamine).

Thus, examples of the polymerisable derivatives of specific binding materials, provided in accordance with the invention, are derivatives of biotin containing a biotinyl group and a polymerisable vinyl group. One very useful and preferred example of such a derivative is the biotin ester of 2-hydroxyethyl methacrylate (HEMA), (corresponding in structure to the esterification of the free carboxyl group of biotin with the 2-hydroxy group of the hydroxyethyl group of HEMA).

If desired, the polymerisation process can be carried out in the presence of high-m.w. materials, e.g. proteins such as enzymes, such as an oxidase or peroxidase enzyme, e.g. glucose oxidase or horseradish peroxidase, which may be desired to be occluded in the polymer. The polymer can be given a degree of crosslinking sufficient to prevent their escape, by suitable choice and quantity of a crosslinking comonomer in the comonomer mixture. The polymerisation process can be performed by any known suitable polymerisation technique, e.g. the vinyl derivatives can be polymerised in deoxygenated aqueous solution in the presence of persulphate and a tertiary diamine such as tetramethylethylenediamine.

In another example, a steroid linked to a polymerisable monomer, e.g. a reaction product of acryloyl chloride with a steroid, can be copolymerised with other acrylic monomer(s) in toluene solution with for example t-butyl hydroperoxide as initiator.

The polymeric products according to the invention need not be solid beadlets. In the first place, polymeric particles made in accordance with the invention can be presented in the form of coatings or carried layers fixed to macro-solid phase carriers such as (larger) beads (e.g. 3-5mm in diameter for microtitre-scale work), or sticks, strips, pegs, plates or the internal surfaces of reaction containers such as microtitre wells.

In the second place, the polymeric products can themselves be formed as coatings or superficial layers or graft copolymers on a substrate of any of the above kinds (such as a substrate polymer) which can itself lack specific binding activity and/or which can lack occluded enzyme activity or antibody activity.

In the third place, the polymeric products need not be solid at all, but can be water-soluble or water-dispersible and made as or converted into aqueous solutions or dispersions, for use as soluble or disperse specific binding materials of multiple specific binding valency.

If desired, the polymeric materials can be of heterogeneous specific binding valency, with each polymer unit, e.g. soluble molecule or solid particle, possessing relatively few, and may be as few as one, of one kind of valency, and relatively many of another kind of valency. For example, a soluble polyacrylamide copolymerised with a monomer mixture including biotinyl-HEMA can possess many biotin groups, or derivatives thereof, or other specific binding groups, convertible if desired to specific binding groups of different and possibly plural specificity by reaction with appropriate conjugates of avidin or streptavidin, or other binding partner as may be appropriate, and it can also be provided with derivatisable groups of different character, may be as few as one per polymer, by for example initiating the polymerisation process with an initiator such as the thermal initiator azobiscyanopentanoic acid, which leaves a carboxyl terminus in the polymeric product. This carboxy-group can be derivatised with protein in known manner, e.g. by coupling in known manner to an antibody.

The result can be used as a highly-useful multiply-labelled antibody for many of the purposes to which labelled antibodies are applied. The labels in such a case can be derived by coupling the biotinyl or other specific binding residues to avidin derivatives (or other complementary binding partner derivatives) of enzymes, radioactively-labelled materials, fluorescent-labelled materials, drug molecules, other proteins, antigens or antibodies or other coupling partners as may be desired.

An example of the preparation and use of materials according to embodiments of this invention is given below by way of illustration and not limitation.

Example:

1.    Preparation of a polymerisable biotin derivative

The 2-hydroxyethyl methacrylate ester of biotin can be prepared by the following process:-

(a)    Add 10ml of thionyl chloride to 1g of d-biotin (from Sigma Chemical Co. (Trade Mark)), keeping the temperature in the range 0-5°C.  Leave for 1 hour with occasional shaking.  Filter off the precipitate formed, and separate any unprecipitated product from the filtrate by adding 20ml of a 1:1 toluene-petroleum ether (100-120°) mixture. Wash the combined precipitates with a further 20ml of the solvent mixture.

(b)    Use the biotinyl chloride product for the next stage immediately.  Store any unused product over $P_2O_5$ after vacuum oven drying at 25°C.

Dissolve 0.5g of the biotinyl chloride in a mixture of 42.5g of 2-hydroxyethylmethacrylate (HEMA) and 0.5g pyridine.  Adjust the pH to approximately 7 by the addition of more pyridine.  Stir at 20°C for two hours. Add the mixture to 400ml toluene, and then add 400ml water.  Shake, and then remove the organic layer.  Add a further 400ml water and repeat the extraction.  Dry the organic layer over anhydrous magnesium sulphate.  Remove toluene on a rotary evaporator, keeping the temperature less than 60°C.  Store the product at 0-5°C.  It contains the 2-hydroxyethylmethacrylate ester of biotin (biotin-HEMA ester) monomer in admixture with a substantial amount of HEMA.

2.    Preparation of polymer beads from comonomer mixture including polymerisable biotin derivative, also containing occluded enzyme.

The following description shows use of the biotin-HEMA monomer prepared as described above to prepare copolymers with acrylamide and methylenebisacrylamide in bead form containing (occluded) active glucose oxidase enzyme, and having covalently-attached biotin groups at the surface, derived from the polymerisable biotin derivative comonomer.  The polymer is sufficiently cross-linked to prevent avidin-enzyme (HRPO) conjugates from entering by penetrative diffusion, and to prevent the occluded enzyme from diffusing out, when the beads are suspended in aqueous liquid.

(a)  Dissolve 7g acrylamide and 0.48g methylenebisacrylamide (MBA) in 32ml of sodium acetate buffer (SAB, pH 5.6) and then add 0.6g of the product from the biotin-HEMA monomer preparation.  (SAB contains 2.84ml acetic acid (glacial) + 99ml water, and is adjusted in pH to 5.6 with sodium hydroxide solution, and diluted to 1 litre with water).

Deoxygenate by bubbling nitrogen through the mixture for fifteen minutes.  Then add 3ml of an ammonium persulphate solution (0.4g/ml) and 8ml of a glucose oxidase solution (2mg/ml, in SAB).  Pour the mixture into a 500ml glass bowl containing 350ml n-hexane and 0.9g SPAN 80 detergent (Trade Mark).

Stir the mixture with an overhead stirrer fitted with a "U" shaped glass stirrer contoured to the bottom and sides of the bowl. Adjust the speed to approximately 220 rpm. Stir for one minute after the addition of the monomer mixture. Then increase the speed to about 250 rpm. Add 2.2ml tetramethylenthylenediamine (TEMED) and continue stirring for three minutes. Turn the speed back down to 220 rpm and continue for one hour. (The bowl should be covered during stirring to reduce evaporation of hexane).

Decant off most of the hexane, filter off the beads, and wash with a large volume of SAB. Remove any large lumps of polymer, and store in SAB with a small amount of thimerosal (antimicrobial preservative) at 0-5°C.

Examples of beads prepared according to the above procedure have been found to show swelling (in SAB) of 5.1g/g and glucose oxidase activity equivalent to about $1.3 \times 10^{-4}$ g (enzyme)/g (dry polymer).

(b) An alternative polymer bead preparation, similar to the above but containing a lower proportion of biotin groups, can be made as follows. The procedure is the same as described in part (a) above, except for the following modifications. The monomer mixture is made with 1ml of a solution of the biotin-HEMA monomer product in water $(6.6 \times 10^{-3}$ g/ml) + 7g acrylamide + 0.48g MBA + 32ml SAB. Examples of beads prepared according to this modified procedure have shown a swelling in SAB of 5.3g/g and a glucose oxidase activity equivalent to about $1.1 \times 10^{-4}$ g (enzyme)/g (dry polymer).

Depending on the end-use of the beads, the occluded glucose oxidase enzyme can be substituted by alternative enzymes or reagents, including specific binding material such as any desired antibody, or omitted.

The beads can be of sizes in the range 30-500 microns diameter, or other size range, according to control of the monomer dispersion process in immiscible organic liquid, as described above.

These beads can be used in any process in which it is desired to bind avidin (or streptavidin) or an avidin or streptavidin derivative to a solid phase. In particular, an immunosorbent of specificity chosen according to need can be prepared on the basis of these beads, by adsorbing to them a conjugate of avidin or streptavidin with an appropriate antigen or antibody or specific binding analogue or specific binding competitor of an antigen or antibody of appropriate specificity.

The beads so treated can then be used as the solid phase of an immunosorbent in an immunosorbent immunoassay, e.g. an enzyme-linked or fluorescence immunoassay of the immunosorbent type.

In further variants of the invention, any of the polymerisable monomer derivatives usable in accordance with the invention can be linked to the specific binding agent derivative through an intermediate linker group instead of directly. The linker group can for example be an ether, polyether, ester, polyester, amide or polyamide chain, or can be a derivatised polysaccharide or protein, for example.

It can be seen that the several features described above can be used in any desired combinations and in modifications or variations within the scope of the invention.

## CLAIMS

1. A process for producing a polymeric product with specific binding affinity, for an antigen or antibody or member of another specific binding pair, comprising the steps of (a) forming a covalently-linked reaction product which is a derivative both of (1) a member of a specific binding pair and of (2) a polymerisable monomer, and (b) polymerising the reaction product from step (a) as a minor component of a polymerisable comomomer mixture.

2. A process according to claim 1, wherein the member of the specific binding pair (1) is a steroid, or an antibiotic, or hapten, or biotin.

3. A process according to claim 1 or 2, wherein the polymerisable monomer is an acrylic monomer.

4. A process according to claim 1, 2 or 3, wherein the polymerisation step (b) is carried out by suspension polymerisation of an aqueous comonomer mixture to produce aqueous cross-linked polymer gel.

5. A process according to claim 4, wherein in the aqueous comonomer mixture there is included a further reagent comprising an enzyme and/or an antibody.

6. A polymeric product produced by a process according to any preceding claim, comprising a polymer body with specific binding affinity at its surface for a member of a specific binding pair.

7. A specific binding complex comprising a polymeric product according to claim 6 with specific binding affinity at its surface for a member of a specific binding pair, bound to said member of a specific binding pair.

8. Polymeric products and their preparative methods, uses and complexes, substantially as hereinbefore described with reference to any of the several features of the foregoing description and examples.

## DOCUMENTS CONSIDERED TO BE RELEVANT

| Category | Citation of document with indication, where appropriate, of relevant passages | Relevant to claim | CLASSIFICATION OF THE APPLICATION (Int. Cl ³) |
|---|---|---|---|
| X | GB-A-1 411 386  (PFIZER INC.)  * Page 2, line 70 - page 3, line 60; claims * | 1,3,4, 6 | G 01 N  33/545 |
| A |  | 5,8 |  |
| X | GB-A-2 071 669  (ITALFARMACO S.p.A.)  * Page 1, lines 1-80 * | 1,3 |  |
| A,D |  | 5,6,8 |  |
| Y | DIE ANGEWANDTE MAKROMOLEKULARE CHEMIE, vol. 72, no. 72, October 1978, pages 31-44; L.D. ANGIURO et al.: "Reaction between glycidylmethacrylate and enzymes and copolymerization of the products obtained"  * Pages 31-34 * | 1-8 |  |
| Y | FR-A-2 332 287  (BEHRINGWERKE AKTIENGESELLSCHAFT)  * Page 5, line 8 - page 7, line 11; claims * | 1-8 |  |
| A,D | EP-A-0 077 671  (ORTHO DIAGNOSTIC SYSTEMS INC.)  * Page 9, line 19 - page 11, line 4; claims *  ---     -/- |  |  |

TECHNICAL FIELDS SEARCHED (Int. Cl ³)

G 01 N
C 12 N
C 08 F

The present search report has been drawn up for all claims

| Place of search | Date of completion of the search | Examiner |
|---|---|---|
| THE HAGUE | 10-01-1986 | HITCHEN C.E. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl ³) |
|---|---|---|---|
| A | FR-A-2 288 104 (SJOHOLM et al.)<br><br>* Page 1, line 1 - page 5, line 12; page 14, example 2 - page 15, example 8; claims * | | |
| A | AT-A- 371 606 (CHANDON INVESTMENT PLANNING LTD.)<br>* Whole document * | 1,3-6, 8 | |

TECHNICAL FIELDS SEARCHED (Int. Cl ³)

The present search report has been drawn up for all claims

| Place of search | Date of completion of the search | Examiner |
|---|---|---|
| THE HAGUE | 10-01-1986 | HITCHEN C.E. |